# EUROPÄISCHE PATENTSCHRIFT

(11) **EP 0 766 975 B1**
(45) Veröffentlichungstag und Bekanntmachung des Hinweises auf die Patenterteilung: **07.08.2002**
(21) Anmeldenummer: 96109807.6
(22) Anmeldetag: 19.06.1996
(51) Int. Cl.: A61M 5/34

(54) **Spritze für medizinische Zwecke**
Syringe for medical use
Seringue pour application médicale

(30) Priorität: 06.10.1995 DE 19537163
(43) Veröffentlichungstag der Anmeldung: 09.04.1997
(73) Patentinhaber: Arzneimittel GmbH Apotheker Vetter & Co. Ravensburg, D-88212 Ravensburg (DE)
(72) Erfinder: Vetter, Helmut, 88212 Ravensburg (DE); Otto, Thomas, 88250 Weingarten (DE)
(74) Vertreter: Dziewior, Joachim, Dipl.-Phys. Dr.

(56) Entgegenhaltungen:
- EP-A- 0 052 804
- EP-A- 0 110 709
- EP-A- 0 281 284
- EP-A- 0 307 169
- EP-A- 0 397 951
- EP-A- 0 441 112

## Beschreibung

Die Erfindung betrifft eine Spritze für medizinische Zwecke, mit einem am Zylindermundstück des Spritzenzylinders angesetzten, durch ein Verschlußteil in Form von beispielsweise einem Tip-Cap bis zum Gebrauch der Spritze verschlossenen Nadelansatzstück, das zum Anbringen einer Kanüle eingerichtet ist, wobei das Nadelansatzstück durch eine Überwurfkappe am Zylindermundstück gehalten und dicht mit dem Spritzenzylinder verbunden ist und die Überwurfkappe durch einen aufgeschobenen und an ihr einrastenden Sicherungsring unlösbar am Zylindermundstück fixiert ist, wobei weiter der Sicherungsring eine fest angeschlossene, aber abtrennbar ausgebildete Sicherungskappe trägt, die das Nadelansatzstück sowie das Verschlußteil umschließt und aus einem im wesentlichen hohlzylindrischen Körper besteht, der an seinen dem Spritzenzylinder abgewandten Ende zumindest teilweise durch eine Stirnwand geschlossen ist.

Eine derartige Spritze ist beispielsweise aus der EP 0 397 951 bekannt und hat sich in der Praxis sehr bewährt. Unter gewissen Umständen kann jedoch der Fall eintreten, daß die zwischen dem Sicherungsring und der Sicherungskappe gebildete Sollbruchstelle sich beim Konfektionieren der Spritzen bereits löst bzw. beschädigt wird, wenn nämlich der Sicherungsring unter Druckeinwirkung von der Sicherungskappe her auf das Nadelansatzstück aufgeschoben wird.

EP-A-307 169 zeigt eine Verschlußkappe und einen Sicherungsring , die zwischen sich einen Ringspalt bilden und über mehrere Verbindungsstege verbunden sind.
Zwischen jeweils zwei Verbindungsstegen ist ein Distanzteil so angeordnet, daß der Abstand zu beiden Verbindungsstegen ein beidseitiges freies Drehspiel der Verschlußkappe ermöglicht .
Das freie Ende jedes Distanzteils bildet eine axiale Stützfläche ("abutment block") für die Verschlußkappe.
Die Verschlußkappe ist jedoch direkt auf einen Flüssigkeitsbehälter montiert um diesen dicht zu verschließen und dient nicht als Sicherungskappe, die zusätzlich von außen auf einen schon wasserdicht und steril abschliessenden Verschlusspfropfen des Flüssigkeitsbehälters aufgesetzt wird.

Der Erfindung liegt die Aufgabe zugrunde, eine Spritze der eingangs genannten Art so auszubilden, daß Beschädigungen der eben beschriebenen Art nicht auftreten können.

Diese Aufgabe wird nach der Erfindung dadurch gelöst, daß die Sicherungskappe und der Sicherungsring zwischen sich einen Ringspalt bilden und über mehrere Verbindungsstege miteinander verbunden sind, wobei zwischen jeweils zwei Verbindungsstegen wenigstens ein Distanzteil angeordnet ist, das an der Sicherungskappe oder am Sicherungsring fest angeschlossen ist und dessen freies Ende dem Sicherungsring oder der Sicherungskappe anliegt oder mit geringem Abstand gegenübersteht.

Der durch die Erfindung erreichte Vorteil besteht im wesentlichen darin, daß die Distanzteile während des Aufschiebens des Sicherungsrings auf das Nadelansatzstück die über die Sicherungskappe einwirkende Kraft auf den Sicherungsring übertragen, ohne daß es hierbei zu Belastungen der Verbindungsstege kommt, die zu deren Abreißen führen könnten. Da die Distanzteile jedoch keine feste Verbindung zwischen der Sicherungskappe und dem Sicherungsring bilden, sind für den Halt der Sicherungskappe vor dem Gebrauch der Spritze allein die Verbindungsstege verantwortlich, die so ausgebildet werden können, daß ein ausreichender Halt der Sicherungskappe gewährleistet ist, ohne daß zum Abtrennen der Sicherungskappe übergroße Kräfte aufgewandt werden müßten. Durch die funktionelle Trennung der Aufgaben, nämlich einerseits eine abtrennbare Verbindung zwischen der Sicherungskappe und dem Sicherungsring durch die Verbindungsstege zu schaffen und andererseits eine die Verbindungsstege nicht belastende Kraftübertragung über die Distanzteile zu schaffen, ist eine Optimierung der beiderseitigen Eigenschaften möglich, ohne daß es zu einer gegenseitigen Beeinflussung kommt.

Soweit insbesondere aus Herstellungsgründen zwischen den Distanzteilen und dem angrenzenden Teil, also dem Sicherungsring oder der Sicherungskappe, ein schmaler Spalt erforderlich ist, sieht die Erfindung in bevorzugter Ausführungsform vor, daß die Verbindungsstege längs einer gedachten Schraubenlinie verlaufend schräg ausgerichtet sind. Hierdurch besitzen die Verbindungsstege eine ausreichende Elastizität, die auch dann Beschädigungen sicher ausschließen, wenn unter Anwendung von Druck die Distanzteile zunächst zur Anlage gegen das angrenzende Teil hin verstellt werden.

Weiter ist es im Rahmen der Erfindung von Vorteil, wenn die Verbindungsstege gleichmäßig über den Umfang verteilt angeordnet sind. Es hat sich darüberhinaus als vorteilhaft herausgestellt, wenn die Verbindungsstege sich zum Sicherungsring hin verjüngen. Dabei können die Verbindungsstege vorteilhafterweise eine im wesentlichen pyramidenförmige Gestalt besitzen.

Die Distanzteile sind im Querschnitt zweckmäßigerweise trapezförmig ausgebildet und verjüngen sich zum Sicherungsring hin.

In weiter zweckmäßiger Ausgestaltung der Erfindung ist vorgesehen, daß der Sicherungsring die Überwurfkappe formschlüssig umgreift und in axialer Richtung durch ein zwischen dem Sicherungsring und der Überwurfkappe angreifendes Rastglied gehalten ist. Dabei kann in besonders einfacher Ausgestaltung das Rastglied von einem an der Innenmantelfläche des Sicherungsrings vorgesehenen Vorsprung gebildet sein, der in eine Ringnut der Überwurfkappe greift und diese an ihrem dem Spritzenzylinder zugewandten Ende hintergreift.

Um bei fertig konfektionierter Spritze die Möglichkeit einer Überprüfung zu haben, ob auf dem Nadelansatzstück ein Tip-Cap aufgesetzt ist, sieht die Erfindung vor, daß die Sicherungskappe mit einer stirnseitig angebrachten axialen Bohrung versehen ist, deren lichter Durchmesser kleiner als der Außendurchmesser des Tip-Caps ist. Wenn dabei die Sicherungskappe vorteilhafter Weise mit ihrer Stirnfläche innenseitig dem Tip-Cap anliegt, ist im übrigen gewährleistet, daß das Tip-Cap sich nicht selbsttätig vom Nadelansatzstück lösen kann.

Schließlich kann hierbei weiter vorgesehen sein, daß die Sicherungskappe sich zu ihrem stirnseitigen Ende hin kegelstumpfförmig verjüngt.

Im folgenden wird die Erfindung an einem in der Zeichnung dargestellten Ausführungsbeispiel näher erläutert; es zeigen:

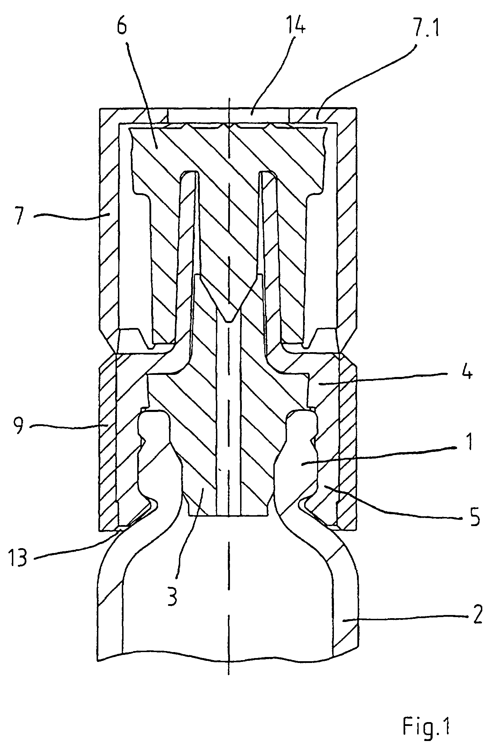

- Fig. 1: eine Spritze nach der Erfindung im Längsschnitt,
- Fig. 2: eine Seitenansicht der Sicherungskappe mit dem Sicherungsring,
- Fig. 3: einen Schnitt durch den Gegenstand nach Fig. 2 längs der Linie A-A.

Die in der Zeichnung nur teilweise dargestellte Spritze findet Verwendung für medizinische Zwecke und besteht aus einem Spritzenzylinder 2 mit einem Zylindermundstück 1, an dem ein Nadelansatzstück 3 angeschlossen ist. Dieses Nadelansatzstück 3 hintergreift mit freigeschnittenen Lamellen 5 das Zylindermundstück 1 und ist bei vorgefüllten Spritzen durch ein Verschlußteil 6 in Form von beispielsweise einem Tip-Cap bis zum Gebrauch verschlossen. Der Spritzenzylinder weist im übrigen in nicht näher dargestellter Weise die übliche Ausgestaltung einer Fingerauflage auf, sowie wenigstens einen durch eine Kolbenstange betätigbaren Stopfen. Bei der Ausbildung als Doppelkammerspritze weist der Spritzenzylinder darüberhinaus einen Bypass sowie einen weiteren Stopfen auf, der vor dem Gebrauch das in lyophilisierter Form eingebrachte pharmazeutische Präparat von dem Lösungsmittel getrennt hält, das vor Anwendung der Spritze über den Bypass in die vordere, kanülenseitige Kammer überführt wird.

Das Nadelansatzstück 3 ist mit einem Konus zum Anbringen einer Kanüle eingerichtet, wobei das Nadelansatzstück 3 durch eine Überwurfkappe 4 am Zylindermundstück 1 gehalten und dicht mit dem Spritzenzylinder 2 verbunden ist. Im Ausführungsbeispiel umgreift die Übewurfkappe 4 das Nadelansatzstück 3 auch im Bereich des Konus.

Die Überwurfkappe 4 ist durch einen aufgeschobenen und an ihr einrastenden Sicherungsring 9 unlösbar am Zylindermundstück 1 fixiert. Der Sicherungsring 9 trägt eine fest angeschlossene, aber abtrennbar ausgebildete Sicherungskappe 7, die das Nadelansatzstück 3 sowie das Tip-Cap 6 umschließt und aus einem im wesentlichen hohlzylindrischen Körper besteht.

Die Sicherungskappe 7 und der Sicherungsring 9 bilden zwischen sich einen Ringspalt 8 und sind über mehrere Verbindungsstege 10 miteinander verbunden. Zwischen jeweils zwei Verbindungsstegen 10 ist jeweils ein Distanzteil 11 angeordnet, das an der Sicherungskappe 7 fest angeschlossen ist und dessen freies Ende dem Sicherungsring 9 mit geringem Abstand gegenübersteht.

Die Verbindungsstege 10 bilden dabei eine Sollbruchstelle, durch die es möglich ist, die Sicherungskappe 7, die die Unversehrtheit der Spritze bis zum Gebrauch gewährleistet, zum Aufsetzen der Kanüle ohne weiteres abzubrechen.

Bei der Konfektionierung der Spritze dagegen, bei der der Sicherungsring 9 auf das Nadelansatzstück 3 bzw. die Überwurfkappe 4 aufgeschoben wird und hierbei die Kraftübertragung über die Sicherungskappe 7 erfolgt, stellen die Distanzteile 11 sicher, daß die Verbindungsstege 10 keine solche Belastung erfahren, daß einzelne oder gar alle abbrechen, da die wesentliche Kraftübertragung über die Distanzteile 11 erfolgt. Die Distanzteile 11 können daher so ausgebildet werden, daß sie eine optimale Kraftübertragung erlauben, während die Verbindungsstege 10 dahingehend optimiert werden können, einerseits einen ausreichenden Halt der Sicherungskappe 7 sicherzustellen, andererseits aber ohne nenneswerte Mühe vor Gebrauch der Spritze abgetrennt werden zu können.

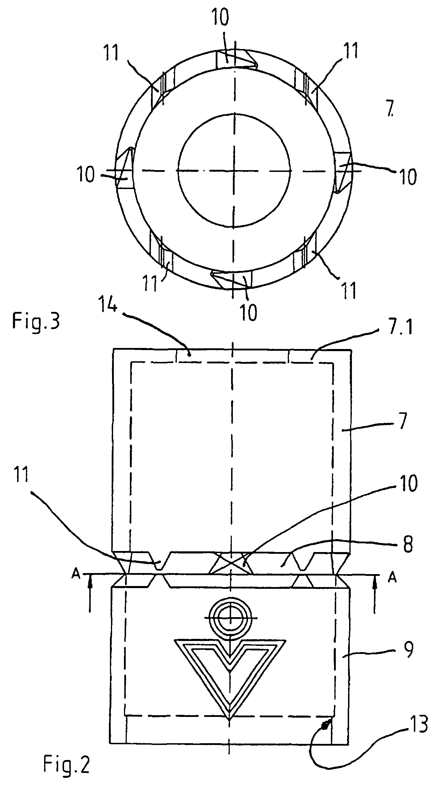

Da es aus herstellungstechnischen Gründen in der Regel nicht gelingt, auf einen Spalt zwischen dem Distanzteil 11 und dem Sicherungsring 9 völlig zu verzichten, sind die Verbindungsstege 10 so ausgerichtet, daß sie längs einer gedachten Schraubenlinie schräg verlaufen. Dabei sind die Verbindungsstege 10 im übrigen gleichmäßig über den Umfang verteilt angeordnet. Wie sich aus der Fig. 2 ersehen läßt, verjüngen sich die Verbindungsstege 10 zum Sicherungsring 9 hin und besitzen eine im wesentlichen pyramidenförmige Gestalt.

Die Distanzteile 11 sind dagegen im Querschnitt trapezförmig ausgebildet und verjüngen sich ebenfalls zum Sicherungsring 9 hin.

Der Sicherungsring 9 umgreift die Überwurfkappe 4 formschlüssig und ist in axialer Richtung durch ein an der Überwurfkappe 4 angreifendes Rastglied 13 gehalten. Dieses Rastglied 13 ist von einem an der Innenmantelfläche des Sicherungsrings 9 vorgesehenen Vorsprung gebildet, der die Überwurfkappe 4 an ihrem dem Spritzenzylinder 2 zugewandten Ende hintergreift.

Um eine Kontrolle der fertig konfektionierten Spritze dahingehend zu ermöglichen, ob das Verschlußteil 6, also das Tip-Cap, auf dem Nadelansatzstück 3 aufgesetzt ist, weist die Sicherungskappe 7 eine stirnseitig angebrachte axiale Bohrung 14 auf, deren lichter Durchmesser aber kleiner als der Außendurchmesser des Tip-Caps 6 ist, so daß dieses nicht vom Nadelansatzstück 3 abgelöst werden kann, solange die Sicherungskappe 7 nicht abgetrennt ist. Um auch ein selbsttätiges Lockern des Tip-Caps 6 zu verhindern, liegt die Sicherungskappe vorteilhafterweise mit ihrer Stirnseite 7.1 innenseitig dem Tip-Cap 6 an. Hierzu kann es im übrigen günstig sein, wenn sich die Sicherungskappe 7 zu ihrem stirnseitigen Ende hin kegelstumpfförmig verjüngt, um für eine Zentrierung des Tip-Caps 6 zu sorgen.

## Patentansprüche

1. Spritze für medizinische Zwecke, mit einem am Zylindermundstück (1) des Spritzenzylinders (2) angesetzten, durch ein Verschlußteil (6) in Form von beispielsweise einem Tip-Cap bis zum Gebrauch der Spritze verschlossenen Nadelansatzstück (3), das zum Anbringen einer Kanüle eingerichtet ist, wobei das Nadelansatzstück (3) durch eine Überwurfkappe (4) am Zylindermundstück (1) gehalten und dicht mit dem Spritzenzylinder (2) verbunden ist und die Überwurfkappe (4) durch einen axial aufgeschobenen und an ihr reibschlüssig einrastenden Sicherungsring (9) unlösbar am Zylindermundstück (1) fixiert ist, wobei weiter der Sicherungsring (9) eine fest angeschlossene, aber abtrennbar ausgebildete Sicherungskappe (7) trägt, die das Nadelansatzstück (3) sowie das Verschlußteil (6) umschließt und aus einem im wesentlichen hohlzylindrischen Körper besteht, der an seiner dem Spritzenzylinder (2) abgewandten Ende zumindest teilweise durch eine Stirnwand (7.1) geschlossen ist, **dadurch gekennzeichnet, daß** die Sicherungskappe (7) und der Sicherungsring (9) zwischen sich einen Ringspalt (8) bilden und über mehrere Verbindungsstege (10) miteinander verbunden sind, wobei zwischen jeweils zwei Verbindungsstegen (10) mit ein beidseitiges freies Drehspiel ermöglichendem Abstand zu diesen wenigstens ein Distanzteil (11) angeordnet ist, das an der Sicherungskappe (7) oder am Sicherungsring (9) fest angeschlossen ist und dessen freies, dem Sicherungsring (9) oder der Sicherungskappe (7) anliegendes oder mit geringem Abstand gegenüberstehendes Ende eine axiale Stützfläche für den Sicherungsring (9) bzw. die Sicherungskappe (7) bildet.

2. Spritze nach Anspruch 1, **dadurch gekennzeichnet, daß** die Verbindungsstege (10) längs einer gedachten Schraubenlinie verlaufend schräg ausgerichtet sind.

3. Spritze nach den Ansprüchen 1 oder 2, **dadurch gekennzeichnet, daß** die Verbindungsstege (10) gleichmäßig über den Umfang verteilt angeordnet sind.

4. Spritze nach einem der Ansprüche 1 bis 3, **dadurch gekennzeichnet, daß** die Verbindungsstege (10) sich zum Sicherungsring (9) hin verjüngen.

5. Spritze nach Anspruch 4, **dadurch gekennzeichnet, daß** die Verbindungsstege (10) eine im wesentlichen pyramidenförmige Gestalt besitzen.

6. Spritze nach einem der Ansprüche 1 bis 5, **dadurch gekennzeichnet, daß** die Distanzteile (11) im Querschnitt trapezförmig ausgebildet sind und sich zum Sicherungsring (9) hin verjüngen.

7. Spritze nach einem der Ansprüche 1 bis 6, **dadurch gekennzeichnet, daß** der Sicherungsring (9) die Überwurfkappe (4) formschlüssig umgreift und in axialer Richtung durch ein zwischen dem Sicherungsring (9) und der Überwurfkappe (4) angreifendes Rastglied (13) gehalten ist.

8. Spritze nach Anspruch 7, **dadurch gekennzeichnet, daß** das Rastglied (13) von einem an der Innenmantelfläche des Sicherungsrings (9) vorgesehenen Vorsprung gebildet ist, der in eine Ringnut der Überwurfkappe (4) greift oder diese an ihrem dem Spritzenzylinder (2) zugewandten Ende hintergreift.

9. Spritze nach einem der Ansprüche 1 bis 8, **dadurch gekennzeichnet, daß** die Sicherungskappe (7) mit einer stirnseitig angebrachten axialen Bohrung (14) versehen ist, deren lichter Durchmesser kleiner als der Außendurchmesser des Tip-Caps (6) ist.

10. Spritze nach einem der Ansprüche 1 bis 9, **dadurch gekennzeichnet, daß** die Sicherungskappe (7) mit ihrer Stirnfläche (7.1) innenseitig dem Tip-Cap (6) anliegt.

11. Spritze nach einem der Ansprüche 1 bis 10, **dadurch gekennzeichnet, daß** die Sicherungskappe (7) sich zu ihrem stirnseitigen Ende hin kegelstumpfförmig verjüngt.

## Claims

1. A syringe for medical purposes, comprising a needle attachment portion (3) which is fitted to the mouthpiece (1) of the syringe cylinder (2) and which is closed by a closure portion (6) in the form for example of a tip cap until the syringe is used and which is designed for mounting a hollow needle, wherein the needle attachment portion (3) is held on the cylinder mouthpiece (1) by a retaining cap (4) and sealingly connected to the syringe cylinder (2) and the retaining cap (4) is non-releasably fixed to the cylinder mouthpiece (1) by a securing ring (9) which is pushed on axially and which is latched thereon in frictional engagement, wherein moreover the securing ring (9) carries a securing cap (7) which is fixedly connected but which is adapted to be separated off and which encloses the needle attachment portion (3) and the closure portion (6) and which comprises a substantially hollow-cylindrical body which, at its end remote from the syringe cylinder (2), is at least partially closed by an end wall (7.1), **characterised in that** the securing cap (7) and the securing ring (9) form an annular gap (8) between them and are connected together by way of a plurality of connecting limbs (10), wherein arranged between each two connecting limbs (10) with a spacing at both sides relative thereto such as to permit free rotational play is at least one spacer portion (11) which is fixedly connected to the securing cap (7) or the securing ring (9) and whose free end which bears against or is in opposite relationship at a small spacing with the securing ring (9) or the securing cap (7) forms an axial support surface for the securing ring (9) or the securing cap (7) respectively.

2. A syringe according to claim 1 **characterised in that** the connecting limbs (10) are oriented to extend inclinedly along a notional helical line.

3. A syringe according to claim 1 or claim 2 **characterised in that** the connecting limbs (10) are arranged distributed uniformly over the periphery.

4. A syringe according to one of claims 1 to 3 **characterised in that** the connecting limbs (10) narrow towards the securing ring (9).

5. A syringe according to claim 4 **characterised in that** the connecting limbs (10) are of a substantially pyramid-shaped configuration.

6. A syringe according to one of claims 1 to 5 **characterised in that** the spacer portions (10) are of trapezoidal cross-sectional configuration and narrow towards the securing ring (9).

7. A syringe according to one of claims 1 to 6 **characterised in that** the securing ring (9) embraces the retaining cap (4) in positively locking relationship and is held in the axial direction by a retaining member (13) which engages between the securing ring (9) and the retaining cap (4).

8. A syringe according to claim 7 **characterised in that** the retaining member (13) is formed by a projection which is provided on the inside peripheral surface of the securing ring (9) and which engages into an annular groove in the retaining cap (4) or which engages behind same at its end which is towards the syringe cylinder (2).

9. A syringe according to one of claims 1 to 8 **characterised in that** the securing cap (7) is provided with an axial bore (14) which is disposed at its end and the inside diameter of which is smaller than the outside diameter of the tip cap (6).

10. A syringe according to one of claims 1 to 9 **characterised in that** the securing cap (7) bears with its end face (7.1) at the inside against the tip cap (6).

11. A syringe according to one of claims 1 to 10 **characterised in that** the securing cap (7) tapers in a frustoconical configuration towards its front end.

## Revendications

1. Seringue à usage médical, comportant un embout de fixation d'aiguille (3), qui est placé sur l'embouchure (1) du cylindre (2) de seringue, est obturé par un élément d'obturation (6), par exemple sous la forme d'un bouchon d'embout, jusqu'à l'utilisation de la seringue et est aménagé pour recevoir une aiguille, l'embout de fixation d'aiguille (3) étant tenu sur l'embouchure (1) du cylindre et étant lié de manière étanche au cylindre (2) de seringue par un capuchon-raccord (4), le capuchon-raccord (4) étant tenu de manière non démontable sur l'embouchure (1) du cylindre de seringue par une bague de fixation (9) enfilée axialement, retenue par adhérence, la bague fixation (9) portant une capuchon de sécurité (7) solidaire mais séparable, qui entoure l'embout de fixation d'aiguille (3) et l'élément d'obturation (6) et est formé d'un corps essentiellement cylindrique creux, dont l'extrémité éloignée du cylindre (2) de seringue est au moins partiellement fermée par une paroi frontale (7.1), **caractérisée en ce que** le capuchon de sécurité (7) et la bague de fixation (9) définissent entre eux un espace annulaire (8) et sont liés l'un à l'autre par plusieurs cloisons de liaison (10), au moins un élément d'espacement (11) étant disposé entre respectivement deux cloisons (10) avec une distance par rapport à celles-ci qui permet un libre jeu en rotation des deux côtés, élément d'espacement qui est solidaire du capuchon de sécurité (7) ou de la bague de fixation (9) et dont l'extrémité libre en contact avec le capuchon de sécurité (7) ou avec la bague de fixation (9), ou disposée à faible distance en vis-à-vis de celui-ci, forme une surface d'appui axiale pour le capuchon de sécurité (7) ou la bague de fixation (9).

2. Seringue selon la revendication 1, **caractérisée en ce que** les cloisons de liaison (10) sont orientées suivant une hélice imaginaire inclinée.

3. Seringue selon la revendication 1 ou 2, **caractérisée en ce que** les cloisons de liaison (10) sont réparties régulièrement sur le pourtour.

4. Seringue selon une des revendications 1 à 3, **caractérisée en ce que** les cloisons de liaison (10) se rétrécissent en direction de la bague de fixation (9).

5. Seringue selon la revendication 4, **caractérisée en ce que** les cloisons de liaison (10) ont essentiellement une forme de pyramide.

6. Seringue selon une des revendications 1 à 5, **caractérisée en ce que** les éléments d'espacement (11), en section transversale, ont une forme de trapèze et se rétrécissent en direction de la bague de fixation (9).

7. Seringue selon une des revendications 1 à 6, **caractérisée en ce que** la bague de fixation (9) entoure avec complémentarité de formes le capuchon-raccord (4) et est retenu dans la direction axiale par une partie d'encliquetage (13) qui agit entre la bague de fixation (9) et le capuchon-raccord (4).

8. Seringue selon la revendication 7, **caractérisée en ce que** l'élément d'encliquetage (13) est formé par une saillie sur la surface périphérique intérieure de la bague de fixation (9) qui pénètre dans une gorge annulaire du capuchon-raccord (4) ou s'engage sous celui-ci à son extrémité tournée vers le cylindre de seringue (2).

9. Seringue selon une des revendications 1 à 8, **caractérisée en ce que** le capuchon de sécurité (7) est muni d'un trou (14) axial aménagé dans sa face frontale, dont le diamètre libre est inférieur au diamètre extérieur du bouhon d'embout (6).

10. Seringue selon une des revendications 1 à 9, **caractérisée en ce que** le capuchon de sécurité (7) est en appui avec sa face frontale (7.1) côté intérieur sur le bouchon d'embout (6).

11. Seringue selon une des revendications 1 à 10, **caractérisée en ce que** le capuchon de sécurité (7) se rétrécit en tronc de cône en direction de son extrémité frontale.
